# EUROPEAN PATENT SPECIFICATION

(11) **EP 0 418 939 B1**
(45) Date of publication and mention of the grant of the patent: **03.11.1993**
(21) Application number: 90202073.4
(22) Date of filing: 27.07.1990
(51) Int. Cl.: C07C 211/50, C07C 209/78, C07C 251/20, C07C 249/02

(54) **Preparation of 9,9-bis(aminophenyl)fluorenes or 9-phenylimidofluorenes**
Herstellung von 9,9-bis(Aminophenyl)fluorenen oder 9-Phenylimidofluorenen
Préparation de bis(aminophényl)-9,9 fluorènes ou de phénylimido-9 fluorènes

(30) Priority: 28.07.1989 US 386080
(43) Date of publication of application: 27.03.1991
(73) Proprietor: SHELL INTERNATIONALE RESEARCH MAATSCHAPPIJ B.V., 2596 HR Den Haag (NL)
(72) Inventor: Li, Simon Ming-Kung, Houston, Texas 77083 (US)

(56) References cited:
- US-A- 4 684 678
- BEILSTEINS "Handbuch der organischen Chemie", 4th edition, supplement I, vol. 1-12, 1933, Verlag von Julius Springer, Berlin, DE, page 176

## Description

It is known to prepare 9,9'-bis(aminophenyl)fluorene by reacting amines with 9-fluorenones in the presence of hydrochloric acid or other strong acids, such as methanesulphonic acid, e.g., in U.S. patent 4,684,678. High temperatures are required for the reaction to produce good yields of products. These high temperatures can make the use of liquid catalysts, such as hydrochloric acid and methanesulphonic acid, more corrosive and difficult to handle in that the acid must be neutralized or washed out before the desired product is recovered. The consistency of the product could also suffer.

Accordingly, there is a need for new, effective catalysts which could be more suitable for use under the practical operating conditions of the process.

The present invention is concerned with the preparation of 9,9'-bis(aminophenyl)fluorenes or 9-iminophenylfluorenes by reacting a 9-fluorenone with a primary or secondary aniline in the presence of an acidic perfluorosulphonic acid polymer catalyst.

The catalysts for the process of the invention are acidic perfluorinated polymers. Such polymers include those perfluorinated polymers that are prepared by conventional polymerization procedures. The acidic perfluorinated polymer usually comprises a copolymer of an ethylenically unsaturated monomer, e.g., an alpha olefin, such as tetrafluoroethylene, ethylene, propylene or the like, and perfluorinated monomers containing acid or precursor acid groups. The resulting copolymers contain preferably from 0.5 to 50 mole percent of perfluorinated sulphonic acid groups. Suitable perfluorinated polymers include those disclosed in U.S. patents 4,303,551, 4,544,458 and 4,626,553.

The perfluorinated polymers are preferably supported on a solid substrate. Suitable supports include organic or inorganic solids, including metallics, such as aluminium, monel, nickel, titanium, copper, brass, stainless steel (Hasteloy), tantalum, silicon carbide, glass, asbestos, zirconium, sulphonyl fluoride polymer, polyolefin, polyamide, polyester polymers, ceramic materials and the like and can optionally contain catalytically active metals, metal oxides, metal ions, metal chelates or other compounds which are catalytically active forms of the above-mentioned metals. Alumina or silicon carbide supports are preferred.

A preferred embodiment of the perfluorinated polymers of the invention is directed to perfluorinated polymers prepared by polymerizing at least two monomers, one of which is ethylenically unsaturated and the other is represented by a perfluorinated monomer as disclosed in U.S. patent 4,330,654. For example, the solid perfluorinated polymer catalyst contains a repeating structure selected from the group consisting of: 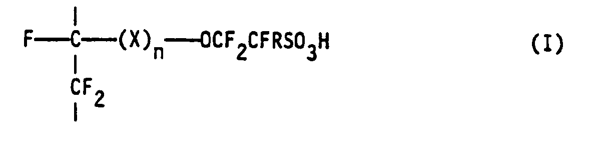
or 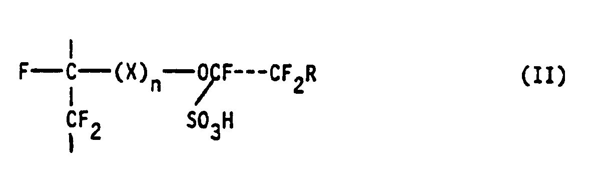
where n is 0, 1 or 2; R is a radical selected from the group consisting of fluorine and perfluoroalkyl radicals having from 1 to 10 carbon atoms; and X is selected from the group consisting of:

(O[CF₂]ₘ), (OCF₂CFY), or (OCFYCF₂)

where m is an integer from 2 to 20 and Y is a radical selected from the class consisting of fluorine and trifluoromethyl radical.

A preferred copolymer prepared by polymerizing perfluoroethylene with a perfluorovinyl ether containing attached sulphonic acid groups has a repeating structure of the formula III: 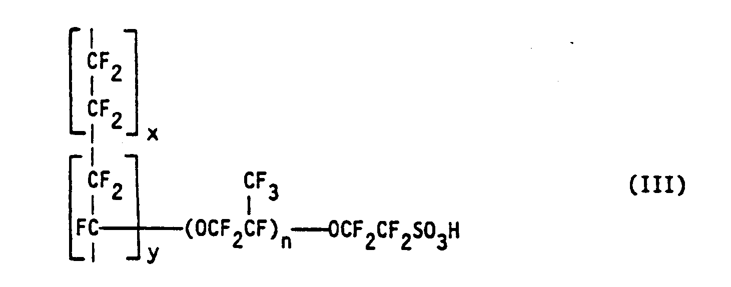
wherein n = 1 or 2 and the ratio of x over y varies from 2 to 50. Catalysts of the above-noted structure III offer the advantages of high concentrations of accessible acid groups in a solid phase.

Suitable 9-fluorenone compounds are known in the art and include those unsubstituted or substituted by one or more substituents on one or both of the aromatic rings by one or more of halogen, alkyl containing 1 to 6 carbon atoms, phenyl, acetyl and the like. Preferably, 9-fluorenone is used.

The precise catalytic amount of the perfluorinated acidic polymer to be used will usually vary to some degree depending on the specific polymer, feed and conditions used for the process. By way of illustration, the catalyst can be present from 0.05 kg per kg of feed per hour to 10.0 kg per kg of feed per hour and preferably from 0.2 kg to 2 kg per kg of feed per hour.

Suitable primary and secondary anilines include those of formula IV 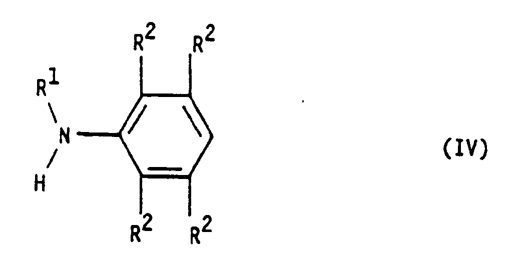
wherein R¹ is hydrogen, an alkyl, cycloalkyl, aryl or aralkyl group containing up to 10 carbon atoms, OH or NO₃; and each R² is independently H, an alkyl group containing 1 to 4 carbon atoms or a halogen atom having an atomic number of 9 to 53. For example, the aniline is N-methylaniline, 2-methylaniline, 2,6-dimethylaniline and the like.

The ratio of the 9-fluorenone to aniline can vary. Usually an excess of aniline is desirable, generally from 2.0 to 20 moles per mole of 9-fluorenone compound can be used, preferably from 5 to 15 moles of aniline per mole of 9-fluorenone compound.

The reaction between the 9-fluorenone compound and the aniline is usually conducted at elevated temperatures. Suitable temperatures are from 120 °C to 250 °C at ambient to slightly elevated pressures up to 4 bar. Preferably, the reaction temperature is from 140 °C to 200 °C.

When secondary amines are used as a reactant the primary product is an amine. When primary amines are used as a reactant, the primary product is a ketimine. While not required, the reaction can take place in the presence of an inert solvent or diluent such as aromatic hydrocarbons, ethers and the like.

The bis(aminophenyl)fluorenes are useful in the synthesis of epoxy resins, such as those disclosed in U.S. patent 4,684,678. The iminophenylfluorenes can be used as blocking agent intermediates to the amino compounds, curing agents or crosslinking agents for various polymers.

Those of skill in the art will appreciate that the process can be conducted as batch, continuous or semi-continuous process. The solid catalyst can be used as a slurry with the reactants in batch process or in a fixed-bed continuous process. The process can use equipment operated in series or in parallel.

### Examples

### Preparation of 9,9'-Bis(N-methylamino-phenyl)fluorene.

One part of the commercial Nafion catalyst (ex Dow Chemical) XU-40036.01 perfluorosulphonic acid polymer supported on alumina was added to a 3.5-part mixture of one part of 9-fluorenone with 10 parts of N-methylaniline heated to 345 °C. By-product water condensed on the top portion of the reaction flask but did not azeotrope as the reaction progressed. The resulting product mixture was separated from the catalyst by filtration and excess N-methylaniline was evaporated to give off-white crystals which were recrystallized from isopropanol to give the desired product as a white solid, m.p. 206 °C and an H-NMR verification of the presence of the amine proton.

### Preparation of 9-(2,6-dimethylimino-phenyl)fluorene.

One part of Nafion XU-40036.01 perfluorosulphonic acid polymer supported on alumina was added to a 3.5-part mixture of one part of 9-fluorenone with 10 parts of 2,6-dimethylaniline heated to 310 °C. By-product water formed and azeotroped off as the reaction progressed. After 6 hours, the reaction product mixture was separated from the catalyst and excess 2,6-dimethylaniline was evaporated off to give bright yellow crystals, which on recrystallization from isopropanol gave the above-named imine, m.p. 121 °C and an absence of amine -NH₂ proton by H-NMR analysis.

## Claims

1. A process for the preparation of 9,9'-bis(aminophenyl)fluorenone or 9-iminophenylfluorene which comprises reacting a 9-fluorenone with a primary or secondary aniline in the presence of an acidic perfluorosulphonic acid polymer catalyst.

2. The process according to claim 1 wherein the perfluorinated polymer catalyst is a copolymer of an ethylenically unsaturated monomer and a perfluorinated monomer containing acid or precursor acid groups.

3. The process according to claim 2 wherein the perfluorinated polymer catalyst has a repeating structure of the formula 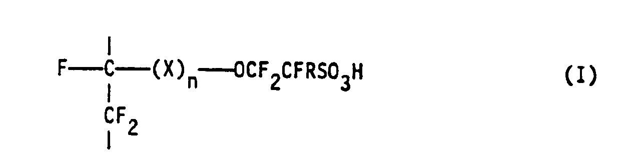 or 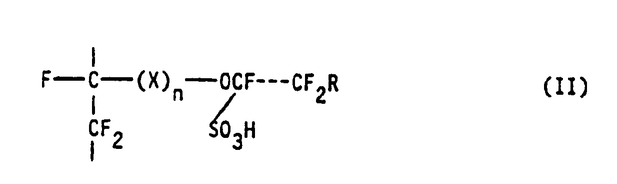 wherein n is 0, 1 or 2; R is a radical selected from the group consisting of fluorine and perfluoroalkyl radicals having from 1 to 10 carbon atoms; and X is selected from the group consisting of:
(O[CF₂]ₘ), (OCF₂CFY), or (OCFYCF₂)
where m is an integer from 2 to 20 and Y is a radical selected from the class consisting of fluorine and the trifluoromethyl radical.

4. A process according to any one of claims 1 to 3 wherein the polymer is prepared by polymerizing perfluoroethylene with a perfluorovinyl ether containing sulphonic acid groups and has a repeating structure of the formula III 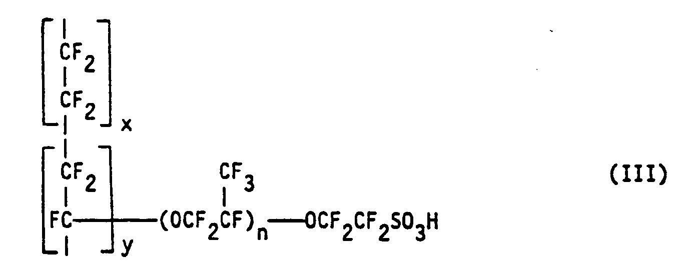 wherein n = 1 or 2 and the ratio of x over y varies from 2 to 50.

5. The process according to any one of claims 1 to 4 wherein the polymer catalyst is a supported catalyst comprising an alumina or silicon carbide support.

6. The process according to any one of claims 1 to 5 wherein the aniline has the formula IV 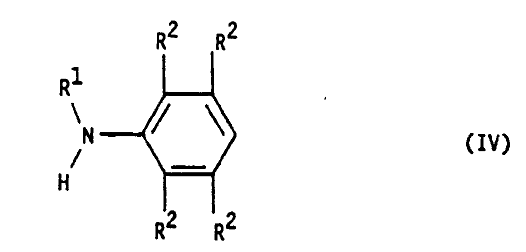 wherein R¹ is hydrogen, an alkyl, cycloalkyl, aryl or aralkyl group containing up to 10 carbon atoms, OH or NO₃; and each R² is independently H, an alkyl group containing 1 to 4 carbon atoms or a halogen atom having an atomic number of 9 to 53.

7. The process according to claim 6 wherein the aniline is N-methylaniline, 2,6-dimethylaniline or 2-methylaniline.

## Patentansprüche

1. Ein Verfahren zur Herstellung von 9,9'-Bis(aminophenyl)fluorenon oder 9-Iminophenylfluoren, das das zur Reaktion bringen eines 9-Fluorenons mit einem primären oder sekundären Anilin in Gegenwart von einem sauren Perfluorsulfonsäurepolymerkatalysator umfaßt.

2. Das Verfahren nach Anspruch 1, wobei der perfluorierte Polymerkatalysator ein Copolymer eines äthylenisch ungesättigten Monomers und eines perfluorierten Monomers, das Säure- oder Vorläufersäuregruppen enthält, ist.

3. Das Verfahren nach Anspruch 2, wobei der perfluorierte Polymerkatalysator eine sich wiederholende Struktur der Formel 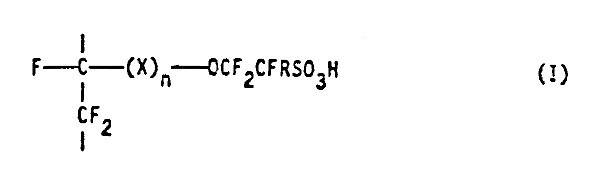 oder 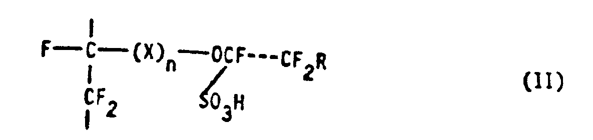 hat, wobei n 0, 1 oder 2 ist; R ein Radikal ist, das aus der Gruppe ausgewählt ist, die aus Fluor- und Perfluoroalkylradikalen besteht, die 1 bis 10 Kohlenstoffatome aufweisen; und X aus der Gruppe ausgewählt ist, die aus
(O[CF₂]m), (OCF₂CFY) oder (OCFYCF₂)
besteht, wobei m eine ganze Zahl von 2 bis 20 und Y ein Radikal ist, das aus der Klasse ausgewählt wurde, die aus dem Fluor- und dem Trifluormethylradikal besteht.

4. Ein Verfahren nach irgendeinem der Ansprüche 1 bis 3, wobei das Polymer hergestellt wird, indem man Perfluoräthylen mit einem Perfluorvinyläther polymerisiert, der Sulfonsäuregruppen enthält und eine sich wiederholende Struktur der Formel III aufweist, 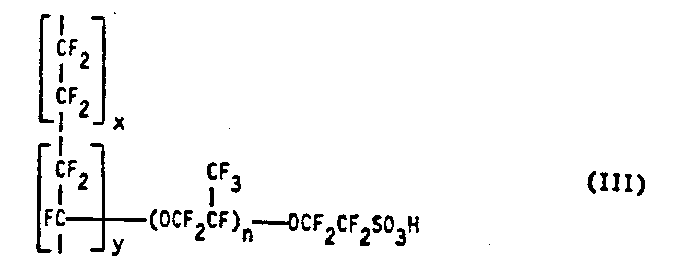 wobei n = 1 oder 2 ist und das Verhältnis von x zu y von 2 bis 50 variiert.

5. Das Verfahren nach irgendeinem der Ansprüche 1 bis 4, wobei der Polymerkatalysator ein Katalysator mit Träger ist, der einen Aluminiumoxid- oder Siliziumkarbidträger umfaßt.

6. Das Verfahren nach irgendeinem der Ansprüche 1 bis 5, wobei das Anilin die Formel IV hat, 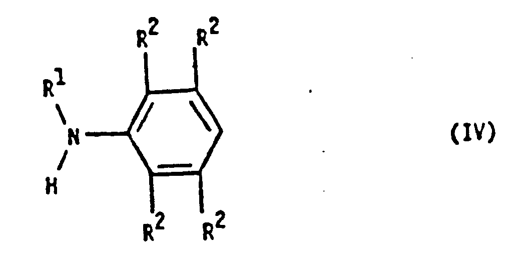 wobei R¹ Wasserstoff, eine Alkyl-, Cycloalkyl-, Aryl- oder Aralkylgruppe, die bis zu 10 Kohlenstoffatome enthält, OH oder NO₃ ist; und jedes R² unabhängig voneinander H, eine Alkylgruppe, die 1 bis 4 Kohlestoffatome enthält, oder ein Halogenatom mit einer Ordnungszahl von 9 bis 53 ist.

7. Das Verfahren nach Anspruch 6, wobei das Anilin N-Methylanilin, 2,6-Dimethylanilin oder 2-Methylanilin ist.

## Revendications

1. Un procédé pour la préparation de 9,9'-bis-(aminophényl)fluorénone ou de 9-iminophénylfluorène, qui comprend la réaction d'une 9-fluorénone avec une aniline primaire ou secondaire en présence d'un catalyseur polymère acide dérivé d'un acide perfluorosulfonique.

2. Un procédé selon la revendication 1, dans lequel le catalyseur polymère perfluoré est un copolymère d'un monomère éthyléniquement insaturé et d'un monomère perfluoré contenant des groupes d'acide ou de progéniteur d'acide.

3. Un procédé selon la revendication 2, dans lequel le catalyseur polymère perfluoré a une maille de la formule 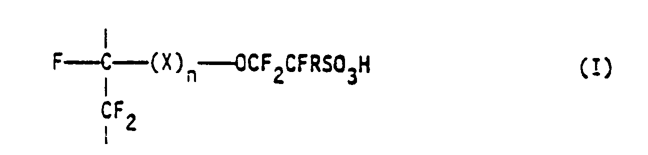 ou 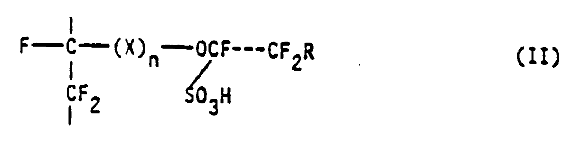 où n est 0, 1 ou 2 ; R est un radical choisi dans le groupe constitué par le fluor et les radicaux perfluoroalcoyle ayant de 1 à 10 atomes de carbone ; et X est choisi dans le groupe constitué par :
(O(CF₂)ₘ), (OCF₂CFY) ou (OCFYCF₂)
où m est un nombre entier de 2 à 20 et Y est un radical choisi dans la classe constituée par le fluor et le radical trifluorométhyle.

4. Un procédé selon l'une quelconque des revendications 1 à 3, dans lequel le polymère est préparé en polymérisant du perfluoroéthylène avec un oxyde de perfluorovinyle contenant des groupes acide sulfonique et a une maille de la formule III 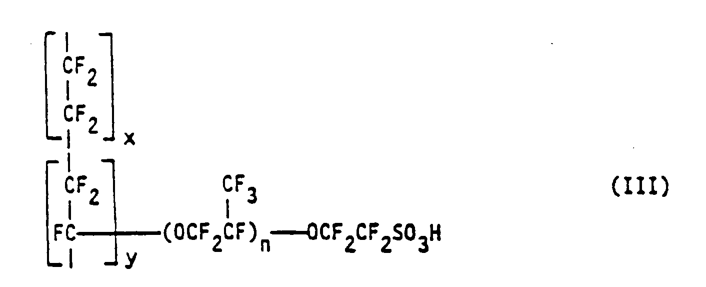 où n = 1 ou 2 et le rapport x:y varie de 2 à 50.

5. Un procédé selon l'une quelconque des revendications 1 à 4, dans lequel le catalyseur polymère est un catalyseur supporté comprenant un support d'alumine ou de carbure de silicium.

6. Un procédé selon l'une quelconque des revendications 1 à 5, dans lequel l'aniline a la formule IV 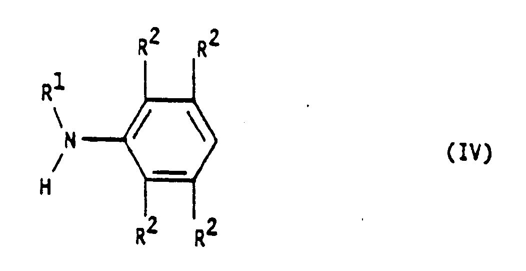 où R¹ est de l'hydrogène ou un groupe alcoyle, cycloalcoyle, aryle ou aralcoyle contenant jusqu'à 10 atomes de carbone, OH ou NO₃ ; et chaque R² est indépendamment H, un groupe alcoyle contenant 1 à 4 atomes de carbone ou un atome d'halogène ayant un nombre atomique de 9 à 53.

7. Un procédé selon la revendication 6, dans lequel l'aniline est la N-méthylaniline, la 2,6-diméthylaniline ou la 2-méthylaniline.
